# EUROPEAN PATENT SPECIFICATION

(11) **EP 0 817 824 B1**
(45) Date of publication and mention of the grant of the patent: **21.11.2001**
(21) Application number: 96907972.2
(22) Date of filing: 29.03.1996
(51) Int. Cl.: C11B 1/00

(54) **PROCESS FOR EXTRACTING AND PURIFYING LIGNANS AND CINNAMIC ACID DERIVATIVES FROM FLAXSEED**
VERFAHREN ZUR GEWINNUNG UND REINIGUNG VON LIGNANEN UND ZIMTSÄUREDERIVATE VON FLACHS-SAATGUT
PROCEDE D'EXTRACTION ET DE PURIFICATION DE LIGNANES ET DE DERIVES DE L'ACIDE CINNAMIQUE A PARTIR DE GRAINES DE LIN

(30) Priority: 31.03.1995 US 415050
(43) Date of publication of application: 14.01.1998
(73) Proprietor: Her Majesty in Right of Canada, represented by The Minister of Agriculture and Agri-Food Canada, Ottawa, Ontario K1A OC5 (CA)
(72) Inventor: WESTCOTT, Neil, D., Saskatoon, Saskatchewan S7H 4A5 (CA); MUIR, Alister, D., Saskatoon, Saskatchewan S7S 1B1 (CA)
(74) Representative: Matthews, Derek Peter
(86) International application number: PCT/CA96/00192
(87) International publication number: WO 96/30468

(56) References cited:
- US-A- 5 484 595
- CHEMICAL ABSTRACTS, vol. 119, no. 3, 19 July 1993 Columbus, Ohio, US; abstract no. 26895, WILSON T.C. ET AL: "Supercritical fluid extraction of polar materials from animal feeds and plant materials" page 762; column 2; XP002021633 & NATL. MEET. - AM. CHEM. SOC. DIV. ENVIRON. CHEM, vol. 33, no. 1, 1993, pages 391-394,
- LEBENSMITTEL-WISSENSCHAFT UND-TECHNOLGIE, vol. 26, no. 4, 1993, LONDON UK, pages 312-317, XP000610486 B. DAVE OOMAH AND G. MAZZA: "Processing of Flaxseed Meal: Effect of Solvent Extraction on Physicochemical Characeristics"
- JOURNAL OF THE AMERICAN OIL CHEMISTS' SOCIETY, vol. 71, no. 6, 1 June 1994, pages 603-607, XP000450510 VARGA T K ET AL: "SIMULTANEOUS EXTRACTION OF OIL AND ANTINUTRITIONAL COMPOUNDS FROM FLAXSEED"

## Description

### Technical Field

This invention relates to an improved process for extracting lignans and cinnamic acid derivatives from flaxseed.

### Background Art

Flaxseed is presently grown for its oil content for use primarily as an industrial oil. In addition, flax is a rich source of fatty acids and has increasing uses in foods. Flaxseed suffers from the fact that the level of fatty acid unsaturation in the triglyceride oil is high and is subject to oxidative polymerization. The scientific literature contains an abundance of reports on the chemistry and physical properties of various components derived from flaxseed. For example, much is known about the feed value of expeller cake meal and its solvent extracted version. The industrial properties of linseed oil are legendary for use in linoleum and paint products. There are also a variety of reports available that describe the extraction and isolation of flaxseed polysaccharides and some suggested uses. Recently, there has been considerable interest shown in a class of minor compounds contained in flaxseed collectively referred to as lignans.

The lignans are generally cinnamic acid dimers containing a dibenzylbutane skeleton. When part of the human diet, certain lignans are believed to be converted into mammalian lignans known as enterolactone and enterodiol. In a study carried out by Thompson et al. (1991) "Mammalian lignan production form various foods", Nutr. Cancer 16: 43-52, 68 different primary foodstuffs were surveyed for their ability to produce mammalian lignans. The results revealed that on a per se basis, whole flaxseed flour and its defatted meal were the highest mammalian lignan producers, the meal and flour being 75 times higher than the next ranking entry, a seaweed, and over 100 times greater than most common foodstuffs. The principal lignan found in flaxseed is secoisolariciresinol diglycoside, referred to hereinafter as SDG.

Flaxseed also contains cinnamic acid derivatives which are distinct from the SDG. The principal cinnamic acid derivatives found in flaxseed are glycosides of p-coumaric acid, ferulic and caffeic acid.

There is considerable published evidence to indicate that lignans exhibit broad spectrum biological activities that include antitumor, antimitotic, antioxidant, antiviral, weak estrogenic and anti-estrogenic activities. Studies within the chemotherapy program of the National Cancer Institute in the United States indicate that certain of the lignans prevent the growth of tumors. Cinnamic acids have known antioxidant properties.

Thompson et al. (1994), "Anticarcinogenic effect of a mammalian lignan precursor from flaxseed", Proc. 55th Flax Institute of the U.S.A., Fargo, ND, 46-50, have studied the effects of SDG on tumorigenesis in rats. When fed in the diet at the promotion stages of tumor formation, SDG can significantly reduce the number of tumors per tumor-bearing rat or per number of rats in the group. At the later stages of mammalian tumorigenesis in rats, SDG can reduce the growth rate of established tumors and the size and number of new tumors formed. Although whole flaxseed or its ground counterpart can be incorporated into foods, the amount or level of usage is restricted by regulation. Furthermore, even if consumption were not regulated, the rather high oil content and the mucilage component of the flax would contribute to excessive caloric intake and excessive laxation respectively.

There are also significant quantities of cyanogenic glycosides present in flaxseed and there is concern regarding excessive consumption of these compounds, as described by Oomah et al. (1992) "Cyanogenic compounds in flaxseed", J. Agric. Food Chem. 40: 1346-1348. Thus, there is also a need to be able to obtain the desirable components of flaxseed without undesirable ones, such as the cyanogenic glycosides.

In a paper by Bakke and Klosterman entitled "A new diglycoside from Flaxseed", Proceedings of the N. Dakota Academy of Science 10: 18-22, (1956), a process is described for extracting SDG from defatted flaxseed using equal parts of 95% ethanol and 1,4-dioxane. Prior publications also refer to methanolysis of complexed SDG (a "polymer") and to the use of sodium or barium methoxide for methanolysis to release non-complexed SDG.

Harris and Haggerty in an article in Cereal Foods World 38: 147-151 (1993) describe the extraction of SDG from defatted flaxseed powder using supercritical fluid carbon dioxide or pure methanol as extraction medium.

In US 5,484,595, Ikeya et al. also describe an extraction system for a particular lignan using etheral or hydrocarbon solvents. This is followed by fractionation and chromatography.

Even though there would appear to be very significant commercial uses for SDG, cinnamic acid glycosides and related derivatives in foods and medicines, the greatest problem has been that even though they have been known for more than 40 years, they are still essentially laboratory curiosities because they are available in only very small quantities. To date, no satisfactory commercial process has been developed for the extraction of lignans, and particularly SDG, from flaxseed. It is, therefore, the main object of the present invention to provide an improved process for extracting SDG and cinnamic acid glycosides from flaxseed which can make them available in not only in highly pure form and in large quantities, but also inexpensively.

### Disclosure of the Invention

The present invention in its broadest aspect relates to a process for extracting lignans (SDG), cinnamic acid glycosides and related derivatives from flaxseed in which a substantially oil-free flaxseed is contacted with a solvent to extract phenolics into the solvent and the phenolic-rich extract obtained is further processed to liberate the lignans and cinnamic acid derivatives therefrom,characterised in that the solvent to extract phenolics comprising secoisolariciresinol diglycoside (SDG) and/or cinnamic acid derivatives is an aqueous aliphatic alcohol solvent, residual solids are separated from the phenolic-rich solvent extract and the phenolic-rich solvent extract obtained is subjected to a base-catalysed hydrolysis to liberate SDG and cinnamic acid derivatives therefrom in a non-complexed form, fractionating the hydrolysed extract into a fraction containing SDG and a fraction containing cinnamic acid derivatives and subjecting either or both said fractions to chromatographic separation to thereby obtain SDG and/or cinnamic acid derivatives at a purity of greater than 95%.

There are various ways for carrying the extractions process. For instance the phenolic-rich solvent may be subjected to the base-catalyzed hydrolysis in an anhydrous alcohol system. An aqueous alcohol system, however, has been found to be particularly advantageous because it is easier and cheaper to operate. It also has the additional unexpected advantage that it destroys the unwanted cyanogenic glycosides present in the flaxseed.

The hydrolyzed extract obtained from the dry alcohol is subjected to a liquid/liquid partition to further enrich the phenolics. The phenolic-rich solution from the aqueous hydrolysis is subjected to contact with an anion exchange resin, or a C-18 solid phase extraction resin, to further separate and enrich the phenolics (lignans and cinnamic acid derivatives). The thus enriched fractions are finally subjected to chromatographic separation to isolate SDG and cinnamic acid derivatives in powdered forms at purities of greater than 95%.

### Best Modes For Carrying Out the Invention

The aliphatic alcohols for the initial extraction which are used in form of an aqueous aliphatic alcohol solvant may be selected from the group consisting of methanol, ethanol, propanol, isopropanol and butanol. A mixture of alcohol and water is surprisingly superior in its extraction capability when compared to the alcohols themselves. Alcohol mixed with up to 50% water gives the best blend for extraction. Thus, the minimum alcohol concentration is preferably at least 50% with an alcohol content of 55 to 75% being particularly preferred.

Methanol has been found to be the most effective solvent in terms of extraction efficiency. Aqueous ethanol is also highly effective and is preferred from considerations of safety to health and handling.

The alcohol solvent is preferably mixed with the flaxseed meal in a liquid to solids ratio in the range 2:1 to 50:1, with a range of 4 - 30:1 being particularly preferred. The contact time between the meal and the solvent system is typically in the range of 1 to 24 hours, with a range of 1 to 6 hours being preferred and a range of 1 to 4 hours being most preferred.

While the extraction temperature is not critical, it is most commonly carried out at a temperature between 0°C and a temperature not exceeding the boiling point of the solvent system of choice. It has been found particularly convenient to carry out the extraction at temperatures between about 20° and 60°C

Following the extraction step, the slurry obtained is subjected to any suitable means of liquid/solid separation as known to those skilled in the art, such as centrifugation, screening, decantation, etc. The residual solids may be further washed with fresh solvent to maximize extraction and the combined liquors may then be filtered by means known to those skilled in the art to produce a liquor free of any suspended solids.

Assays of crude extracts have shown that neither SDG, cinnamic acid glycosides, or their corresponding alkyl esters are present in a free form. In order to isolate the SDG and cinnamic acid derivatives in good yield, it is necessary to subject the extract to a base-catalyzed hydrolysis. This hydrolysis may be of either anhydrous or aqueous form.

### (a) Anhydrous system

For the anhydrous system, the extract is concentrated to remove solvent by any of a variety of means known to those skilled in the art, e.g. evaporation under reduced pressure, and dried to a generally anhydrous state which may be a thick, syrupy material, e.g. by a technique such as rotary evaporation, freeze-drying, drum drying, spray drying, etc. The hydrolysis may then be carried out using a dry alcohol, e.g. dry ethanol or methanol together with a hydrolyzing agent such as sodium methoxide and possibly barium methoxide. This hydrolyzing agent may be added in a typical amount of 0.06-2.5% w/v based on the total volume of liquid. This results in the liberation of lignan plus esters of the corresponding alcohol and other agents which can be difficult to remove. It has also quite surprisingly been found that triethylamine may be effectively used for hydrolysis, e.g. in amounts up to 1.25% w/v based on the total volume of liquid. While it may be possible to use organic amines other than triethylamine, it has been found that triethylamine has important advantages. Thus, it is a medium strength base with a boiling point sufficiently high as not to cause volatility problems during hydrolysis while still being sufficiently low to be removed by traditional evaporative procedures following hydrolysis.

The anhydrous alcohol hydrolysate is subjected to liquid/liquid partitioning and this can be conveniently carried out in a continuous extractor using an ethyl acetate/water solvent system with the ethyl acetate and water preferably being in a ratio in the range of about 1:1 to 7:1. Ethyl acetate is particularly preferred for this purpose because of its polarity. Thus, solvents with polarity below ethyl acetate are unable to effectively remove non-lignan substances from aqueous solutions, which is essential for effective subsequent chromatographic purification of SDG. The non-lignan substances removed in the ethyl acetate fraction may include valuable products including derivatives of cinnamic acid glycosides; e.g. methyl-coumarate 4-glycoside, methyl caffeate 4-glycoside, and methyl ferulate 4-glycoside.

Isolation of SDG at high purity is preferably accomplished by reverse-phase chromatography. Thus, the SDG-rich fraction from the liquid/liquid partitioning may be further purified by contacting a reverse-phase resin, e.g. a slurry or column of C-18 or C-8 reverse phase packing. Examples of the C-18 and C-8 reverse phase packing include Radial-Pak® C-18, Bondapak® C-18, Bondapak® C-B and Nova-Pak® C-18, all available from Waters. After the SDG-rich eluant has contacted the resin, the resin is washed with a dilute acid, e.g. 1% acetic acid, to remove organic and inorganic substances and then eluted with acidic aqueous alcohol, e.g. 1% acetic acid in aqueous methanol, to provide an enriched SDG fraction.

### (b) Aqueous system

For the aqueous base hydrolysis, the extract does not need to be in an anhydrous form and may be used in the form of a concentrated syrupy residue. The hydrolysis may also be performed in the aqueous alcohol extract without prior concentration of the extract. The base is usually selected from ammonium hydroxide, sodium hydroxide and potassium hydroxide, with sodium or potassium hydroxide being preferred. The concentration of the base is typically about 1 normal and it is preferably used in an amount of about 3-7% w/v. The hydrolysis is normally carried out over about 4 to 24 hours with the shortest possible hydrolysis time preferred. An additional benefit of this process is the destruction of the cyanogenic glycosides yielding an extract free of cyanogenic glycosides or free cyanide.

In one embodiment of the aqueous system, the aqueous base hydrolysate contains the SDG, together with other components including cinnamic acid derivatives, and this hydrolysate is contacted with an anion exchange resin. It is necessary to adjust the pH of the hydrolysate to the acidic range, e.g. about pH 3-7, preferably pH 4-6, before contacting the anion exchange resin. This can be done using any suitable inorganic or organic acid, such as hydrochloric acid, sulphuric acid, acetic acid, etc. However, acetic acid is preferred for this purpose.

The anion exchange is preferably carried out in an anion exchange chromatography column prepared in the acetate counterion form. Many different commercially available resins may be used, e.g. A-25 QAE Sephadex® , Q-Sepharose® , DEAE Sephadex® , or Dowex 1® . The acidified hydrolsate is applied to the column and then the column is eluted with water to elute the SDG and some organic and inorganic materials. Further elution of the column with acid, e.g. 50% acetic acid in 15% ethanol elutes acid components which may include valuable products, such as glycosides of cinnamic acids and other cinnamic acid derivatives, e.g. caffeic, ferulic and p-coumaric acid glycosides. When these cinnamic acid glycosides were converted into the corresponding methyl esters by the use of alkylating agents such as diazomethane, the methyl esters obtained proved to be identical to those obtained in the anhydrous hydrolysis procedure.

The column may be conveniently regenerated for use by means of aqueous sodium hydroxide followed by aqueous acetic acid and finally by water.

Isolation of SDG at high purity is accomplished by reverse-phase chromatography as described above for the liquid/liquid partitioning fraction. The aqueous eluate from the anion exchange column is further purified by contacting a reverse-phase resin, e.g. a slurry or column of C-18 or C-8 reverse phase packing as described above. After the SDG-rich eluant has contacted the resin, the resin is washed with a dilute acid, e.g. 1% acetic acid, to remove organic and inorganic substances and then eluted with acidic aqueous alcohol, e.g. 1% acetic acid in aqueous methanol, to provide an enriched SDG fraction.

In another embodiment of the aqueous system, the aqueous base hydrolysate is purified by solid phase extraction chromatography on C-18 reverse-phase resin. The solid phase extraction chromatography is preferably carried out on C-18 reverse-phase resin prepared in dilute aqueous organic acid at a pH of about 3-6, preferably about pH 3-4. This can be done with any organic acid, such as acetic acid, formic acid, etc; however, 1% acetic acid is preferred for this purpose. Although Preparative C-18 125Å resin available from Waters has been found to work well in the process, it is recognized that there are other commercial sources of C-18 resins that work equally well. The acidified hydrolysate is applied to the column and the column eluted with dilute organic acid. This can be done with any organic acid, such as acetic acid, formic acid, etc; however, 1% acetic acid is preferred for this purpose. The SDG and other valuable products including the cinnamic acid glycosides are retained on the column and recovered by elution with alcohol, e.g. methanol or ethanol.

The SDG can be further separated from the other valuable products in the solid phase extraction alcohol eluate by an additional solid phase extraction chromatography step. The alcohol eluate is concentrated to the aqueous phase by any of a variety of means known to those skilled in the art, e.g. evaporation under reduced pressure. This weakly acidic residue is adjusted to pH 7 with base, e.g. sodium hydroxide and contacted with solid phase extraction C-18 reverse-phase resin equilibrated in water. The column or resin slurry is washed with water and dilute aqueous alcohol e.g. less than 10% aqueous methanol or ethanol, to yield a fraction rich in cinnamic acid glycosides. The column or resin slurry is further washed with alcohol, e.g. greater than 50% methanol or ethanol, with greater than 80% alcohol being preferred, to yield a fraction rich in SDG.

The enriched SDG solutions, including the SDG rich alcohol fraction from the solid phase extraction chromatography, may then be further processed by preparative high pressure liquid chromatography (HPLC), for example using a C-18 reverse-phase column support and an elution system consisting of water/acidic acid/methanol under gradient conditions of 100% aqueous acidic acid to 40% aqueous acidic acid/60% methanol. The SDG thus eluted was found to be purified to a level of greater than 95% in terms of physical, spectral and structural analysis consistent with known literature values.

The cinnamic acid glycoside methyl ester rich fraction from the liquid/liquid partitioning, and the cinnamic acid glycoside rich fractions from anion exchange and solid phase extraction processes may be further processed by preparative HPLC on reverse-phase resin using aqueous acetic acid/methanol gradients as for SDG, to obtain eluates with purity levels greater than 95%.

Employing the aqueous base hydrolysis strategy as a screening tool, it has been possible to detect the flaxseed lignan, SDG, in amounts of up to 30 mg per gram of defatted flaxseed. This represents up to a 10 fold increase in concentration over previously known techniques as described in the published literature.

### Brief Description of the Drawings

In the drawings which illustrate the present invention:
Fig. 1 is a flowsheet of one embodiment of the process;
Fig. 2 is a flowchart of a further embodiment of the process;
Fig. 3 is a chromatogram of a triethylamine hydrolysate of an alcoholic extract of flaxseed meal;
Fig. 4 is a chromatogram of a sodium hydroxide hydrolysate of an alcoholic extract of flaxseed meal;
Fig. 5 is a chromatogram of the aqueous (SDG) fraction after ethyl acetate extraction of triethylamine hydrolysate;
Fig. 6 is a chromatogram of the ethyl acetate fraction after ethyl acetate extraction of triethylamine hydrolysate;
Fig. 7 is a chromatogram of the aqueous (SDG) wash after anion exchange of the sodium hydroxide hydrolysate;
Fig. 8 is a chromatogram of the alcoholic wash after anion exchange of the sodium hydroxide hydrolysate;
Fig. 9 is a chromatogram of SDG after HPLC purification;
Fig. 10 is a chromatogram of the alcohol eluate from the solid phase extraction C-18 treatment of the aqueous sodium hydroxide hydrolysate;
Fig. 11 is a chromatogram of the cinnamic acid glycoside fraction recovered from the second solid phase extraction C-18 treatment of the aqueous sodium hydroxide hydrolysate;
Fig. 12 is a chromatogram of the SDG enriched fraction recovered from the second solid phase extraction C-18 treatment of the aqueous sodium hydroxide hydrolysate;
Fig. 13 is a chromatogram of p-coumaric acid 4-glycoside after HPLC purification;
Fig. 14 is a chromatogram of methyl-coumarate 4-glycoside after HPLC purification;
Fig. 15 is a chromatogram of ferulic acid 4-glycoside after HPLC purification and
Fig. 16 is a chromatogram of methyl ferulate 4-glycoside after HPLC purification.

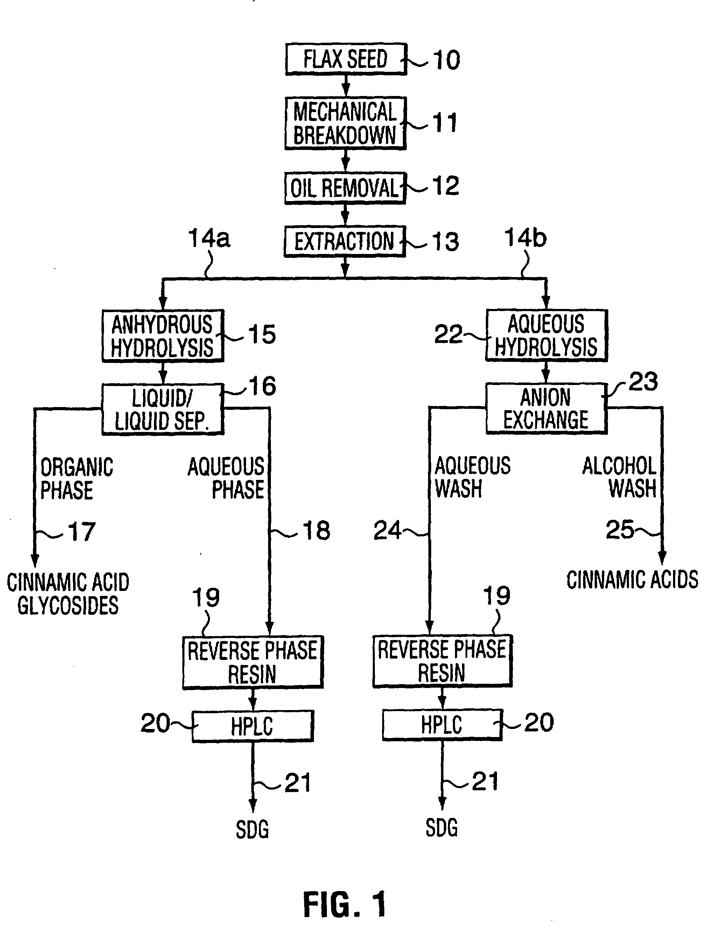

As shown in Figure 1, a supply of flaxseed 10 is mechanically broken down by grinding, flaking or pressing 11 and oil is removed 12. The oil-free meal is then fed to an extraction vessel 13 where it is contacted with aqueous alcohol. The extract obtained is then either fed via line 14a to an anhydrous system or via line 14b to an aqueous system.

In the anhydrous system, the extract 14a is preferably concentrated by removing alcohol to a generally anhydrous state and then fed to a hydrolysis vessel 15 where it is hydrolyzed with triethylamine in dry alcohol. The hydrolyzate obtained is subjected to liquid/liquid separation in a separation vessel 16 using an organic/water solvent system. An organic fraction 17 is obtained containing cinnamic acid derivatives and an aqueous fraction 18 is obtained containing SDG. This SDG-containing fraction is further purified by contacting a reverse phase resin 19 followed by high pressure liquid chromatography (HPLC) 20. The eluate 21 contains SDG at a purity of greater than 95%. In the aqueous system, the extract 14b is fed to a hydrolysis vessel 22 where it is hydrolyzed with sodium hydroxide in aqueous alcohol. The hydrolyzate obtained is passed through an anion exchange chromatography column 23. This column 23 is eluted with water to remove an SDG-containing eluate 24. This eluate was then passed through a reversed phase chromatography column 19 followed by HPLC 20. The eluate 21 contains SDG at a purity of greater than 95%.

The column 23 is also eluted with alcohol to obtain an alcohol eluate 25 containing cinnamic acid glycosides at a purity of greater than 95%.

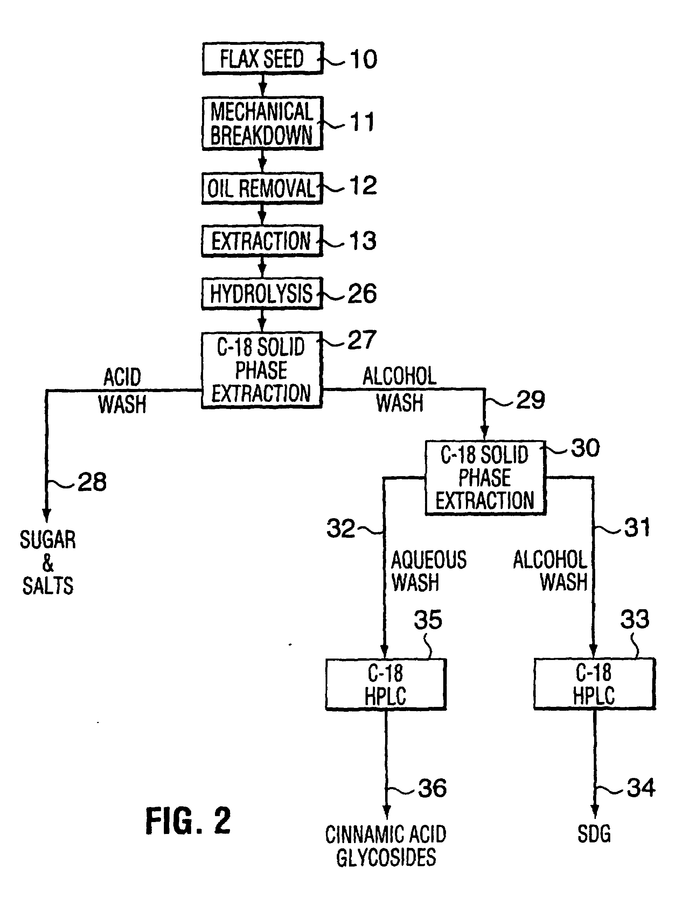

Figure 2 shows an alternative flow sheet in which the initial steps including the extraction 13 are carried in the same manner as in Figure 1. The extract obtained is fed to a hydrolysis vessel 26 in which it is hydrolyzed with sodium hydroxide in aqueous alcohol. The aqueous-base hydrolyzate is contacted with C-18 reverse phase resin 27. Salts and sugars 28 are removed from the resin by elution with an acid solution. The resin is also eluted with alcohol to obtain an alcohol eluate 29. This eluate 29, which is reduced to the aqueous phase is then contacted with a further C-18 reverse-phase resin column 30, which is eluted with water and dilute alcohol. The aqueous eluate is passed through a C-18 HPLC 35 and an eluate 36 is obtained containing cinnamic acid glycosides. The alcohol eluate 31 is passed through a C-18 HPLC 33 and eluate 34 is obtained containing SDG at a purity of greater than 95%. The invention is further illustrated by the following non-limiting examples.

### Example 1

Substantially oil-free flaxseed meal was obtained from CanAmera Foods (Attona, MB.). This meal had been commercially crushed and solvent extracted. A 0.5 kg sample of meal was extracted at room temperature by intermittent slurrying with a mixture of 70% methanol and 30% water in a liquid:solids ratio of 6:1. After 24 hours the suspension was filtered and the extract was evaporated under reduced pressure to produce a syrupy concentrate which was further dried to form a dry extract. The dried extract was then subjected to a base-catalyzed hydrolysis using triethylamine in anhydrous methanol in a ratio of 1:100.

The hydrolysate that was obtained from triethylamine catalysis was then subjected to liquid/liquid partitioning using ethyl acetate/water in the ratio 4:1. The ethyl acetate phase containing methyl esters and other ethyl acetate solubles was concentrated under reduced pressure, and the aqueous phase containing SDG was freed of ethyl acetate by concentration under reduced pressure. The SDG containing fraction was further purified by contacting C-18 reverse-phase resin packed in a low pressure column. After the SDG containing fraction had contacted the resin, the column was washed with an aqueous solution of 1% acetic acid in 30% methanol to remove a SDG enriched fraction that was concentrated under reduced pressure. The purity of SDG as greater than 60% by weight.

The SDG separated fraction was then subjected to preparative scale high pressure liquid chromatography (HPLC) using a column packed with C-18 reverse phase resin, e.g. Radial-Pak® C-18 packing (Waters Chromatography). The eluting system consisted of water/acetic acid/methanol under gradient conditions of 100% aqueous acetic acid to 40% aqueous acetic acid/60% methanol. The eluants contained SDG at a purity of greater than 95%. The SDG was recovered from the eluate by concentration under reduced pressure and finally obtained as a dry powder by freeze-drying.

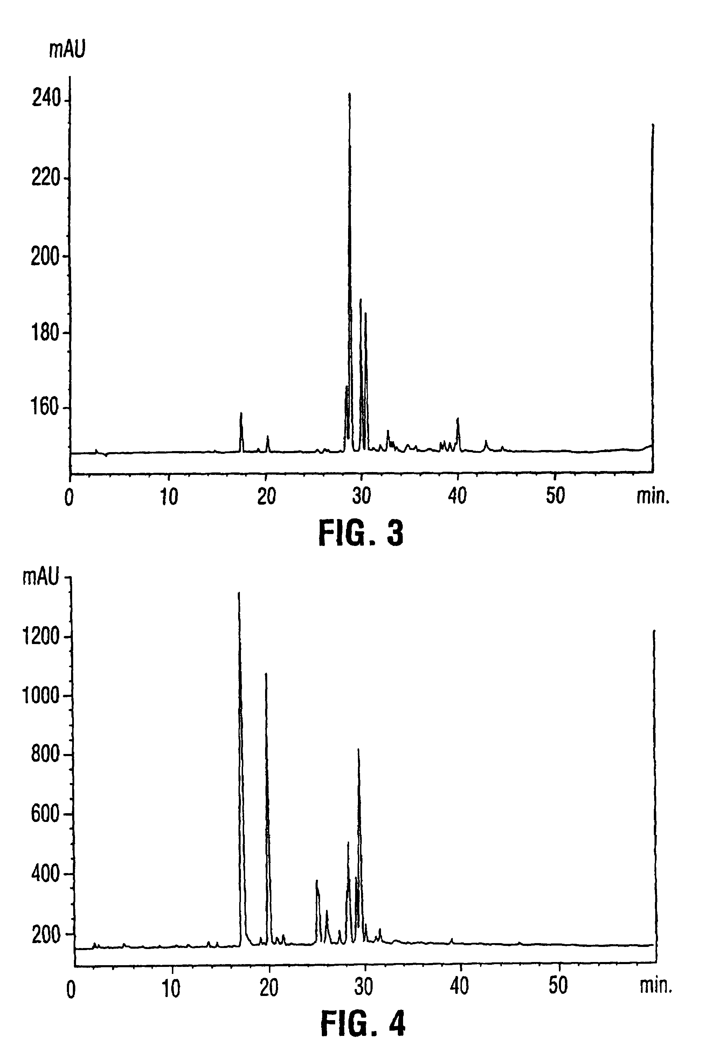

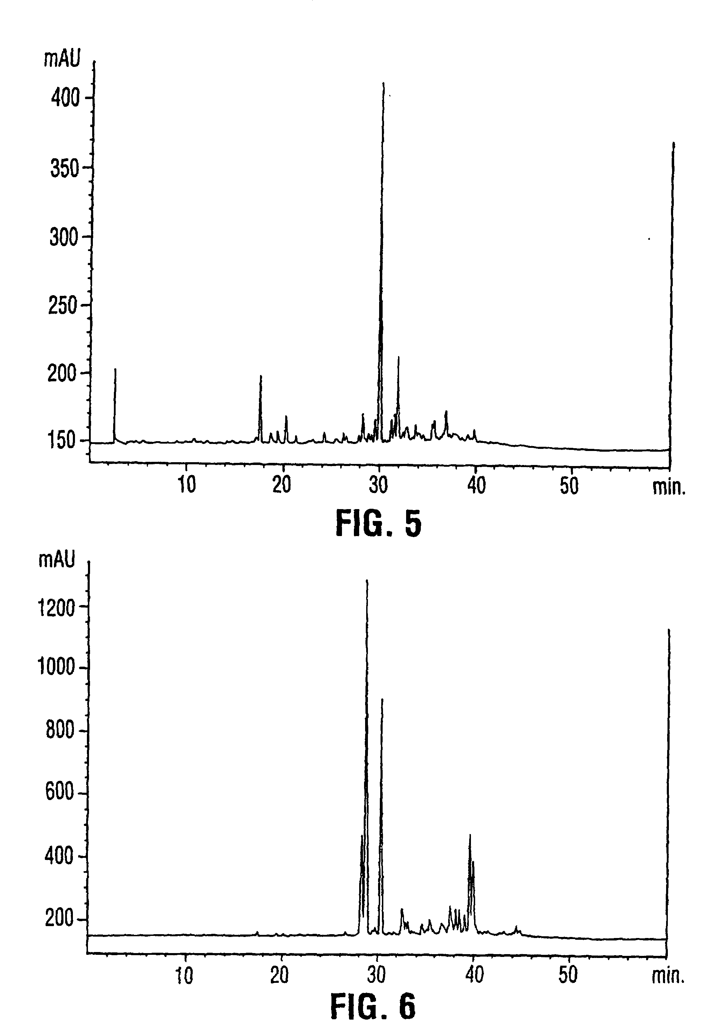

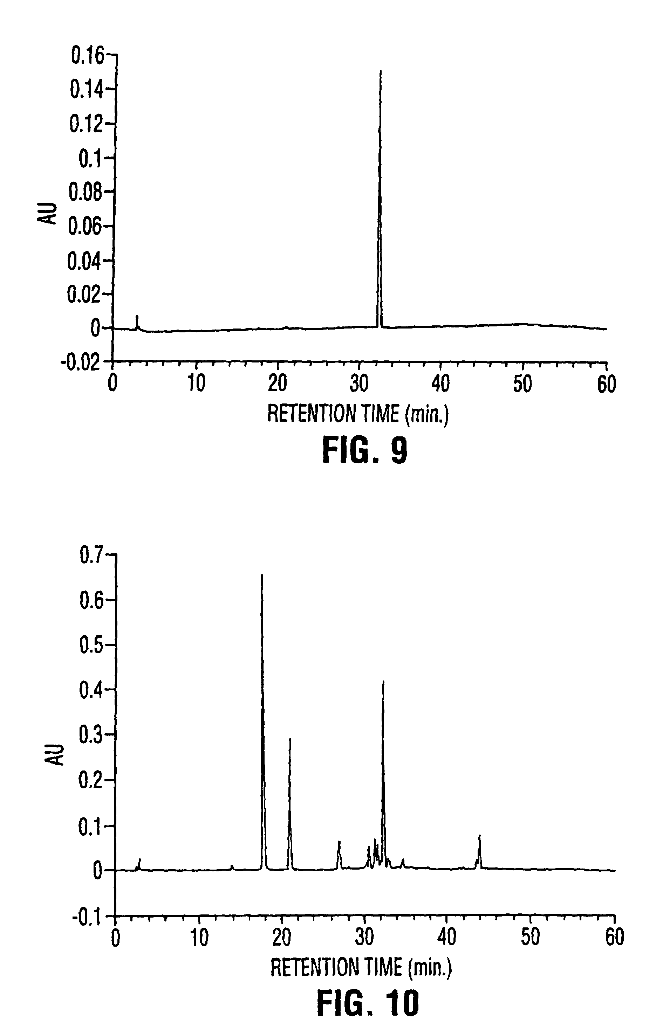

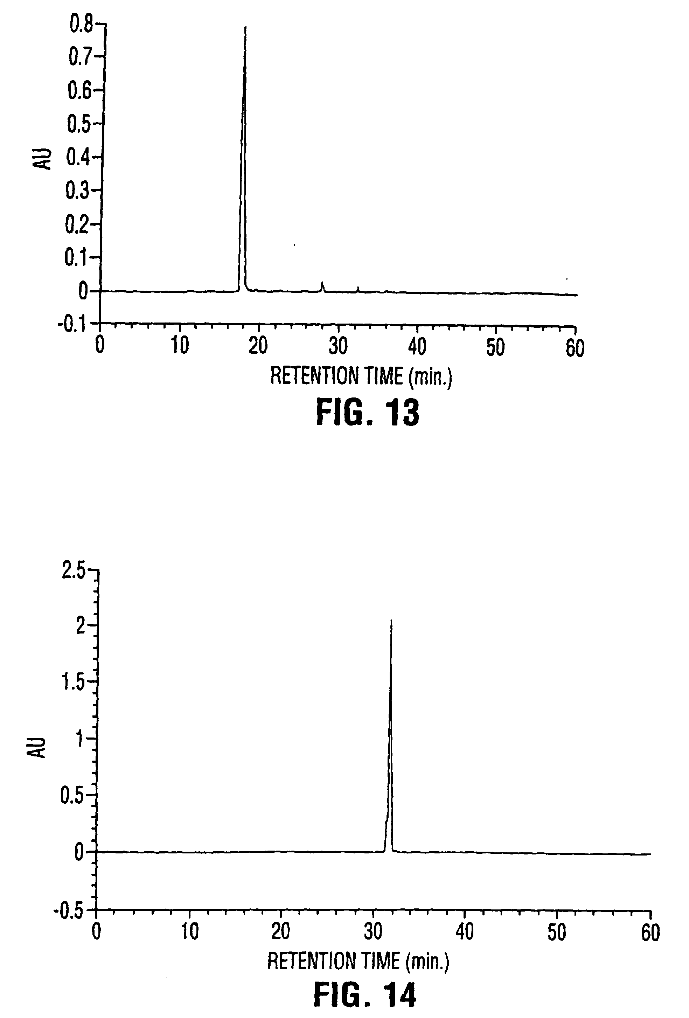

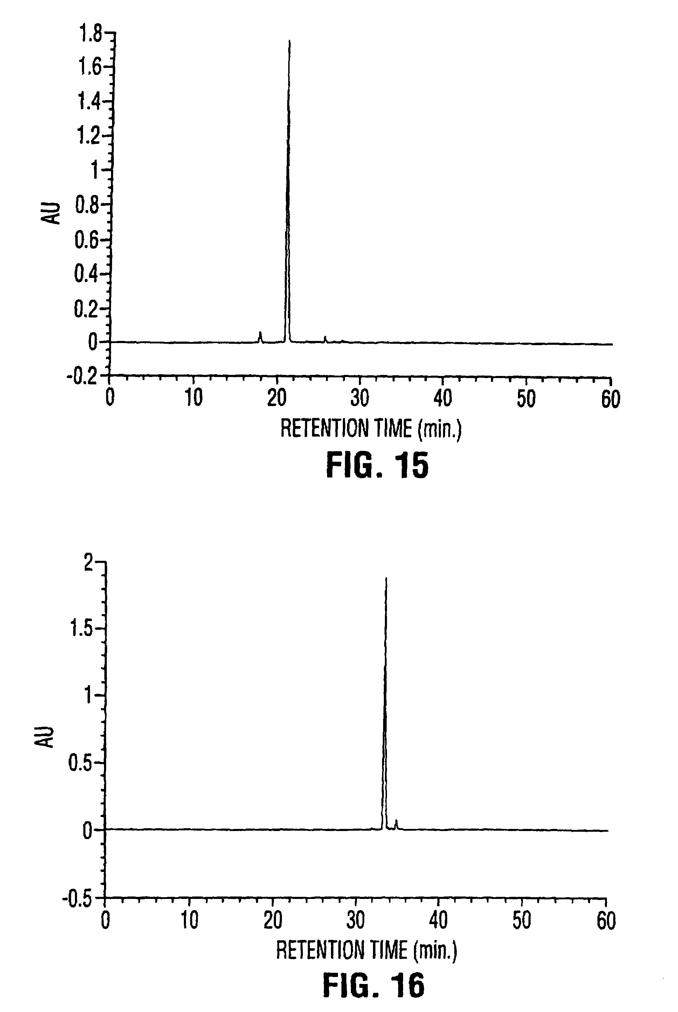

Fig. 3 shows a chromatogram for a triethylamine hydrolyzate, Fig. 5 shows a chromatogram of the aqueous phase after ethyl acetate extraction, Fig. 6 shows the ethyl acetate phase of the extraction, while Fig. 9 shows a chromatogram of the purified SDG. Fig. 14 shows a chromatogram of purified methyl coumarate 4-glycoside, while Fig. 16 shows a chromatogram of purified methyl ferulate 4-glycoside recovered from the ethyl acetate fraction. These show the effectiveness of the ethyl acetate extraction and reverse-phase resins in separating SDG from co-extractives thereby simplifying the isolation and purification,

### Example 2

Substantially oil-free flaxseed meal was obtained from CanAmera Foods (Attona, MB.). This meal had been commercially crushed and solvent extracted. A 0.5 kg sample of meal was extracted at room temperature by intermittent slurrying with a mixture of 70% methanol and 30% water in a liquid:solids ratio of 6:1. After 24 hours the suspension was filtered and the extract was evaporated under reduced pressure to produce a syrupy concentrate.

The concentrate was then subjected to base-catalyzed hydrolysis using sodium hydroxide in water in a ratio of 5:100. The pH of the hydrolysate that was obtained from sodium hydroxide hydrolysis was adjusted to 5 using acetic acid and the aqueous phase so obtained contacted A-25 QAE Sephadex anion exchange resin in a column prepared in the acetate counterion form. Thereafter, the column was eluted with water to elute the SDG and unspecified organic and inorganic substances. The column was then further eluted with 50% acetic acid in 15% ethanol, to obtain an eluate containing acid components, including the glycosides of caffeic, ferulic acid and p-coumaric acid, and other cinnamic acid derivatives.

The SDG containing fraction was further purified using C-18 reverse-phase resin in the same manner as in Example 1. The SDG enriched fraction thus obtained was then subjected to preparative scale high pressure liquid chromatography using a column packed with C-18 reverse-phase resin in the same manner as in Example 1, to obtain eluates containing SDG at a purity of greater than 95%.

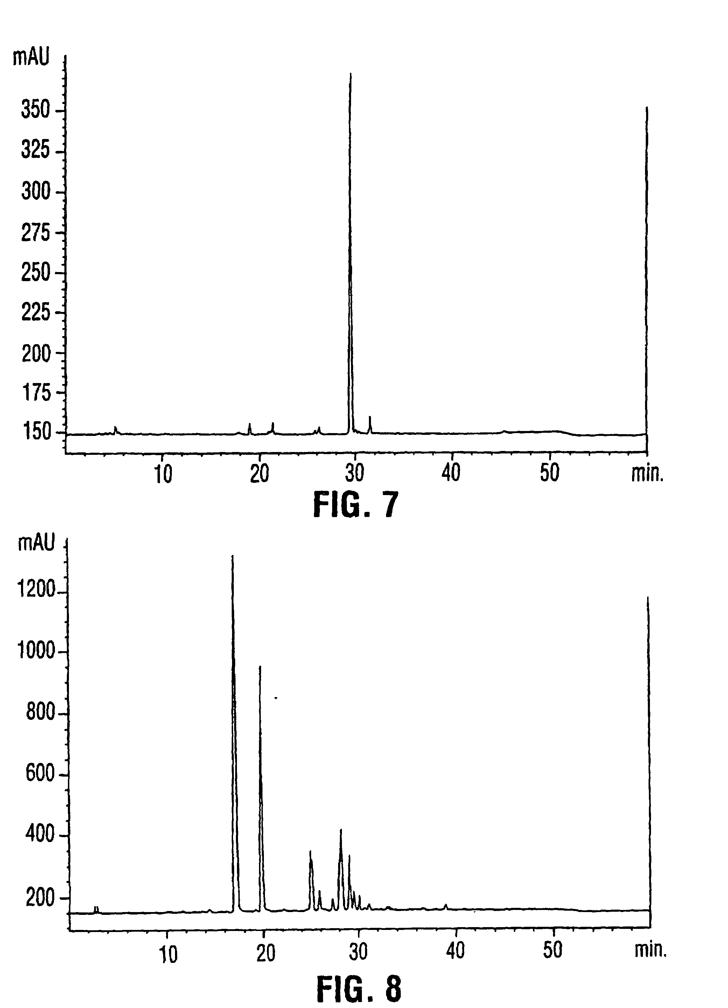

Fig. 4 shows a chromatogram for an aqueous sodium hydroxide hydrolysate, Fig. 7 shows a chromatogram of the aqueous (SDG) wash after anion exchange chromatography of the sodium hydroxide hydrolysate, while Fig. 9 shows a chromatogram of the purified SDG after HPLC purification. Fig. 13 shows a chromatogram of purified p-coumaric acid 4-glycoside, while Fig. 15 shows a chromatogram of purified ferulic acid 4-glycoside. These show the effectiveness of the anion exchange and reverse-phase resins in separating SDG from co-extractives thereby simplifying the isolation and purification.

### Example 3

Substantially oil-free flaxseed meal was obtained from CanAmera Foods (Attona, MB.). This meal had been commercially crushed and solvent extracted. A 2 kg sample of meal was extracted at room temperature with stirring with a mixture of 50% ethanol (w/v) in a liquid:solids ratio of 6.25:1. After 4 hours the suspension was filtered and the solids re-extracted with an additional 15L of 50% ethanol (w/v). The combined extract was evaporated under reduced pressure to produce a syrupy concentrate.

The concentrate was then subjected to base-catalyzed hydrolysis using sodium hydroxide in water in a ratio of 5:100. The pH of the hydrolysate was adjusted to 3 to 4 using acetic acid and the aqueous phase so obtained was contacted with a reverse-phase C-18 resin, e.g. Preparative C-18 125Å (Waters Corporation), equilibrated in 1% acetic acid. Thereafter the resin was washed with 1% acetic acid to remove sugars, salts and unspecified organic and inorganic substances. The resin was further eluted with methanol or ethanol to recover a fraction enriched in SDG and cinnamic acid glycosides.

The SDG containing fraction was further purified by neutralizing the fraction to pH 6-7 with sodium hydroxide pellets or 50% w/v sodium hydroxide, and concentrating the fraction to the aqueous phase. The SDG containing fraction was contacted with a reverse-phase C-18 resin, e.g. Preparative C-18 125Å (Waters Corporation), equilibrated in water. Thereafter the resin was washed sequentially with water and dilute aqueous alcohol to recover the cinnamic acid glycosides. The resin was further eluted with methanol or ethanol to recover a fraction enriched in SDG.

The SDG enriched fraction thus obtained was then subjected to preparative scale high pressure liquid chromatography using a column packed with C-18 reverse-phase resin in the same manner as in Example 1, to obtain eluates containing SDG at a purity of greater than 95%.

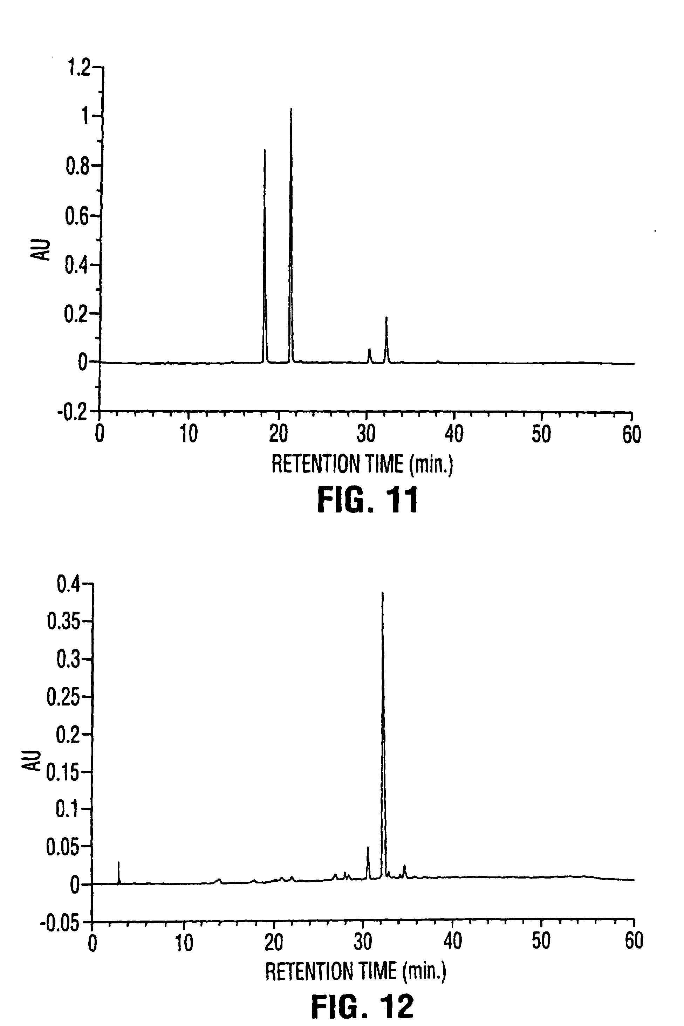

Fig. 10 shows a chromatogram of the alcohol eluate recovered from the first solid phase extraction treatment of an aqueous sodium hydroxide hydrolysate, Fig. 11 shows a chromatogram of the cinnamic acid glycosides present in the water wash of the second solid phase extraction treatment, Fig. 12 shows the SDG enriched alcohol fraction from the second solid phase extraction treatment, while Fig. 8 shows a chromatogram of the purified SDG after HPLC purification. Fig. 13 shows chromatogram of purified p-coumaric acid 4-glycoside, while Fig. 15 shows a chromatogram of purified ferulic acid 4-glycoside. These show the effectiveness of the solid phase extraction treatment and reverse-phase resins in separating SDG and the cinnamic acid glycosides from co-extractives thereby simplifying the isolation and purification.

In the above examples, the chromatograms of Figures 2-7 were obtained on a Hewlett Packard analyzer, while those of Figures 8-16 were obtained on a Waters analyzer.

### Industrial Applicability

The invention can be used as a large scale industrial process for extracting valuable components from flaxseed in commercial quantities.

## Claims

1. A process for extracting lignans and cinnamic acid derivatives from flaxseed wherein a substantially oil-free flaxseed meal is contacted with a solvent to extract phenolics into the solvent and the phenolic-rich extract obtained is further processed to liberate the lignans and cinnamic acid derivatives therefrom,**characterised in that** the solvent to extract phenolics comprising secoisolariciresinol diglycoside (SDG) and/or cinnamic acid derivatives is an aqueous aliphatic alcohol solvent, residual solids are separated from the phenolic-rich solvent extract and the phenolic-rich solvent extract obtained is subjected to a base-catalysed hydrolysis to liberate SDG and cinnamic acid derivatives therefrom in a non-complexed form, fractionating the hydrolysed extract into a fraction containing SDG and a fraction containing cianamic acid derivatives and subjecting either or both said fractions to chromatographic separation to thereby obtain SDG and/or cinnamic acid derivatives at a purity of greater than 95%.

2. A process according to claim 1 wherein the aqueous aliphatic alcohol solvent contains at least 50% by weight of alcohol.

3. A process according to claim 2, wherein the alcohol is methanol or ethanol.

4. A process according to claim 1 or 2, wherein the phenolic-rich alcohol solvent is concentrated by removal of solvent so as to obtain a phenolic extract in dry form.

5. A process according to claim 4, wherein the base-catalysed hydrolysis is carried out by slurrying the dried extract in anhydrous ethanol or methanol and in the presence of sodium alkoxide or triethylamine.

6. A process according to claim 4, wherein the phenolic extract in anhydrous form is subjected to liquid/liquid partition, e.g. using an ethyl acetate/water system.

7. A process according to claim 6, wherein the lignan-rich fraction from the liquid/liquid partition is further enriched in SDG by contacting with a reverse phase resin.

8. A process according to claim 1, wherein the phenolic-rich alcohol extract is concentrated by removal of solvent to form a syrupy concentrate.

9. A process according to claim 8, wherein the base-catalyzed hydrolysis is carried out by mixing the concentrate with aqueous alkali metal hydroxide, e.g. sodium or potassium hydroxide, or ammonium hydroxide.

10. A process according to claim 9, wherein the aqueous base hydrolysate obtained containing SDG is contacted with an anion exchange resin, after which the resin is eluted with water to elute the SDG together with some organic and inorganic materials.

11. A process according to claim 10, wherein the aqueous base hydrolysate is neutralised to a pH in the range 3-7, preferably 4-6, before contacting the anion exchange resin.

12. A process according to claim 11, wherein the eluate containing SDG is further enriched in SDG by contacting with a reverse-phase resin, after which the resin is eluted with a dilute acid/alcohol gradient to obtain SDG at a purity greater than 95%

13. A process according to claim 6, wherein a separate product containing cinnamic acid glycosides and other cinnamic acid derivatives is removed in the ethyl acetate fraction.

14. A process according to claim 10 wherein after the resin is eluted with water to elute the SDG, it is further eluted with dilute acid to remove a separate product containing cinnamic acid glycosides and other cinnamic acid derivatives.

15. A process according to claim 9, wherein the aqueous base hydrolysate obtained containing SDG is contacted with a C-18 reverse-phase solid phase extraction resin, after which the resin is washed with dilute acid, e.g. acetic acid, and eluted with alcohol, e.g. methanol or ethanol to elute the SDG together with some organic materials.

16. A process according to claim 15 wherein the alcohol eluate from the C- 18 reverse-phase solid phase extraction resin is further contacted with a C-18 reverse-phase solid phase extraction resin equilibrated in water, after which the resin is washed with water and dilute alcohol, e.g methanol or ethanol, to recover the cinnamic acid glycosides, and the resin eluted with alcohol or an alcohol water mixture to recover the SDG.

17. A hydrolysed extract of flaxseed meal comprising SDG and cinnamic acid glycosides in a non-complexed form, obtainable by contacting substantially oil-free flaxseed meal with an aqueous aliphatic alcohol solvent to extract phenolics into the solvent, separating residual solids from the phenolic-rich solvent extract and subjecting said phenolic-rich solvent extract to a base-catalysed hydrolysis.

18. A hydrolysed extract of flaxseed meal as claimed in claim 17 additionally free of cyanogenic glycosides.

## Patentansprüche

1. Verfahren zur Extraktion von Lignanen und Zimtsäurederivaten aus Leinsamen, wobei ein im wesentlichen ölfreies Leinsamenmehl mit einem Lösungsmittel in Kontakt gebracht wird, um Phenolverbindungen in das Lösungsmittel zu extrahieren, und der erhaltene phenolreiche Extrakt weiterverarbeitet wird, um die Lignane und die Zimtsäurederivate daraus freizusetzen, **dadurch gekennzeichnet, daß** das Secoisolariciresinol-Diglucosid (SDG) und/oder Zimtsäurederivate aufweisende Lösungsmittel zur Extraktion der Phenolverbindung ein wäßriger aliphatischer Alkohol ist, daß restliche Feststoffe von dem phenolreichen Lösungsmittelextrakt abgetrennt werden und der erhaltene phenolreiche Lösungsmittelextrakt einer basenkatalysierten Hydrolyse unterzogen wird, um die SDG und die Zimtsäurederivate daraus in einer nicht-komplexgebundenen Form freizusetzen, Fraktionieren des hydrolysierten Extrakts in eine SDG enthaltende Fraktion und eine Zimtsäurederivate enthaltende Fraktion und Unterziehen jeder der beiden Fraktionen einer chromatographischen Trennung, um dadurch SDG und/oder Zimtsäurederivate mit einer Reinheit von mehr als 95 % zu erhalten.

2. Verfahren nach Anspruch 1, wobei der wäßrige aliphatische Alkohol mindestens 50 Gewichts-% Alkohol enthält.

3. Verfahren nach Anspruch 2, wobei der Alkohol Methanol oder Ethanol ist.

4. Verfahren nach Anspruch 1 oder 2, wobei das phenolreiche alkoholische Lösungsmittel durch Entfernen des Lösungsmittels konzentriert wird, um einen phenolischen Extrakt in trockener Form zu erhalten.

5. Verfahren nach Anspruch 4, wobei die basenkatalysierte Hydrolyse durch Rufschlämmen des getrockneten Extrakts in wasserfreiem Ethanol oder Methanol und in der Gegenwart von Natriumalkoholat oder Triethylamin durchgeführt wird.

6. Verfahren nach Anspruch 4, wobei der phenolische Extrakt in wasserfreier Form einer Flüssig-/Flüssig-Trennung, z.B. unter Verwendung eines Ethylacetat-/Wasser-Systems, unterzogen wird.

7. Verfahren nach Anspruch 6, wobei die lignanreiche Fraktion aus der Flüssig-Flüssig-Trennung durch Kontaktieren mit einem Umkehrphasenharz weiter mit SDG angereichert wird.

8. Verfahren nach Anspruch 1, wobei der phenolreiche alkoholiache Extrakt durch Entfernen von Lösungsmittel konzentriert wird, um ein sirupartiges Konzentrat zu bilden.

9. Verfahren nach Anspruch 8, wobei die basenkatalysierte Hydrolyse durch Mischen des Konzentrats mit wäßrigem Alkalimetallhydroxid, z.B. Natrium- oder Kaliumhydroxid oder Ammoniumhydroxid, durchgeführt wird.

10. Verfahren nach Anspruch 9, wobei das erhaltene SDG enthaltende wäßrige basische Hydrolysat mit einem Anionenaustauschharz in Kontakt gebracht wird, wonach das Harz mit Wasser eluiert wird, um das SDG zusammen mit einigen organischen und anorganischen Materialien zu eluieren.

11. Verfahren nach Anspruch 10, wobei das waßrige basische Hydrolysat auf einen pH-Wert in den Bereich von 3 bis 7, vorzugsweise von 4 bis 6, neutralisiert wird, bevor es mit dem Anionenaustauschharz in Kontakt gebracht wird.

12. Verfahren nach Anspruch 11, wobei das SDG enthaltende Eluat durch Kontaktieren mit einem Umkehrphasenharz weiter mit SDG angereichert wird, wonach das Harz mit einem Gradienten aus verdünnter Säure und Alkohol eluiert wird, um SDG mit einer Reinheit von mehr als 95 % zu erhalten.

13. Verfahren nach Anspruch 6, wobei ein getrenntes Produkt, das Zimtsäureglucoside und andere Zimtsäurederivate enthält, aus der Ethylacetatfraktion entfernt wird.

14. Verfahren nach Anspruch 10, wobei nachdem das Harz mit Wasser eluiert wurde, um das SDG zu eluieren, es weiter mit verdünnter Säure eluiert wird, um ein getrenntes Produkt zu entfernen, das Zimtsäureglucoside und andere Zimtsäurederivate enthält.

15. Verfahren nach Anspruch 9, wobei das erhaltene SDG enthaltende wäßrige basische Hydrolysat mit einem festen C-18-Umkehrphagenextraktionsharz in Kontakt gebracht wird, wonach das Harz mit verdünnter Säure, z.B. Essigsäure, gewaschen wird und mit Alkohol, z.B. Methanol oder Ethanol, eluiert wird, um das SDG zusammen mit einigen organischen Materialien zu eluieren.

16. Verfahren nach Anspruch 15, wobei das Alkoholeluat von dem festen C-18-Umkehrphasenextraktionsharz darüber hinaus mit einem in Wasser equilibrierten festen C-18-Umkehrphasenextraktionsharz in Kontakt gebracht wird, wonach das Harz mit Wasser und verdünntem Alkohol, z.B. Methanol oder Ethanol, gewaschen wird, um die Zimtsäureglucoside zu erhalten, und das Harz mit Alkohol oder einer Alkohol-Wasser-Mischung eluiert wird, um das SDG zu erhalten.

17. Hydrolyaierter Extrakt aus Leinsamenmehl, der SDG und zimtsäureglucoside in einer nicht-komplexgebundenen Form enthält, das erhältlich ist durch Kontaktieren des im wesentlichen ölfreien Leinsamenmehls mit einem wäßrigen aliphatischen Alkohol, um Phenolverbindungen in das Lösungsmittel zu extrahieren, Abtrennen restlicher Feststoffe aus dem phenolreichen Lösungsmittelextrakt und Unterziehen des phenolreichen Lösungsmittelextrakts einer basenkatalysierten Hydrolyse.

18. Hydrolysierter Extrakt aus Leinsamenmehl nach Anspruch 17, der zudem frei von cyanogenen Glycosiden ist.

## Revendications

1. Procédé d'extraction de lignanes et de dérivés de l'acide cinnamique à partir de graines de lin dans lequel une farine de graines de lin pratiquement exempte de matières grasses est mise au contact d'un solvant de façon à extraire les composés phénoliques dans le solvant et en ce que l'on traite en outre l'extrait riche en composés phénoliques obtenu de façon à en libérer les lignanes et les dérivés de l'acide cinnamique, ledit procédé étant **caractérisé en ce que** le solvant utilisé pour extraire les composés phénoliques comprenant du diglycoside de sécoisolaricirésinol (SDG) et/ou des dérivés de l'acide cinnamique est un solvant de type alcool aliphatique aqueux, **en ce que** les solides résiduels sont séparés du solvant d'extraction riche en composés phénoliques et **en ce que** le solvant d'extraction riche en composés phénoliques obtenu est soumis à une hydrolyse catalysée par une base de façon à libérer le SDG et les dérivés de l'acide cinnamique sous forme non complexée, l'extrait hydrolysé est fractionné en une fraction contenant du SDG et une fraction contenant les dérivés de l'acide cinnamique, et, l'on soumet soit l'une, soit les deux de ces fractions à une séparation par chromatographie de façon à obtenir le SDG et/ou les dérivés de l'acide cinnamique à un degré de pureté supérieur à 95%.

2. Procédé selon la revendication 1, dans lequel le solvant de type alcool aliphatique aqueux contient au moins 50% en poids d'alcool.

3. Procédé selon la revendication 2, dans lequel l'alcool est le méthanol ou l'éthanol.

4. Procédé selon la revendication 1 ou 2, dans lequel le solvant de type alcool riche en composés phénoliques est concentré par élimination du solvant de façon à obtenir l'extrait phénolique sous forme sèche.

5. Procédé selon la revendication 4 dans lequel l'hydrolyse est réalisée en reprenant l'extrait sec dans de l'éthanol ou du méthanol anhydre en présence d'alcoolate de sodium ou de triéthylamine.

6. Procédé selon la revendication 4 dans lequel l'extrait phénolique sous forme anhydre est soumis à une séparation par partage liquide/liquide, par exemple en utilisant un système d'acétate d'éthyle/eau.

7. Procédé selon la revendication 6 dans lequel la fraction riche en lignanes résultant de la séparation par partage liquide/liquide est enrichie ensuite en SDG par mise en contact avec une résine pour phase inverse.

8. Procédé selon la revendication 1 dans lequel l'extrait alcoolique riche en composés phénoliques est concentré par élimination du solvant de façon à former un concentré sirupeux.

9. Procédé selon la revendication 8 dans lequel l'hydrolyse catalysée par une base est réalisée par mélange du concentré avec un hydroxyde de métal alcalin aqueux, par exemple l'hydroxyde de sodium ou de potassium, ou l'hydroxyde d'ammonium.

10. Procédé selon la revendication 9 dans lequel l'hydrolysat basique aqueux obtenu contenant le SDG est mis au contact d'une résine échangeuse d'anions, puis en ce que la résine est éluée avec de l'eau de façon à éluer le SDG ensemble avec quelques matériaux de type organique et minéral.

11. Procédé selon la revendication 10 dans lequel l'hydrolysat basique aqueux est neutralisé à un pH situé dans la plage allant de 3 à 7, de préférence de 4 à 6, avant d'être mis au contact de la résine échangeuse d'anions.

12. Procédé selon la revendication 11 dans lequel l'éluat contenant le SDG est enrichi en outre en SDG par mise au contact avec une résine pour phase inverse, puis la résine est éluée avec un gradient acide dilué/alcool de façon à obtenir le SDG à un degré de pureté supérieur à 95%.

13. Procédé selon la revendication 6, dans lequel un produit séparé contenant des glycosides de l'acide cinnamique et d'autres dérivés de l'acide cinnamique est éliminé dans la fraction d'acétate d'éthyle.

14. Procédé selon la revendication 10 dans lequel après avoir éluer la résine avec de l'eau de façon à éluer le SDG, elle est ensuite éluée avec un acide dilué de façon à éliminer un produit séparé contenant des glycosides de l'acide cinnamique et d'autres dérivés de l'acide cinnamique.

15. Procédé selon la revendication 9, dans lequel l'hydrolysat basique aqueux obtenu contenant le SDG est mis au contact d'une résine C-18 pour phase inverse, d'extraction en phase solide, puis la résine est lavée avec un acide dilué par exemple l'acide acétique et éluée avec de l'alcool, par exemple le méthanol ou l'éthanol de façon à éluer le SDG ensemble avec quelques matériaux organiques.

16. Procédé selon la revendication 15 dans lequel l'éluat alcoolique sortant de la résine C-18 pour phase inverse d'extraction en phase solide est en outre mise au contact d'une résine C-18 pour phase inverse, d'extraction en phase solide équilibrée dans de l'eau, puis la résine est lavée avec de l'eau et de l'alcool dilué, par exemple le méthanol ou l'éthanol, de façon à récupérer les glycosides de l'acide cinnamique, et la résine est éluée avec de l'alcool ou un mélange alcool/eau de façon à récupérer le SDG.

17. Extrait hydrolysé de farine de graines de lin comprenant du SDG et des glycosides de l'acide cinnamique sous forme non complexées, pouvant être obtenu par mise au contact d'une farine de graines de lin pratiquement exempte de matières grasses avec un solvant de type alcool aliphatique aqueux de façon à extraire des composés phénoliques dans le solvant, par séparation des solides résiduels du solvant d'extraction riche en composés phénoliques et par soumission dudit solvant d'extraction riche en composés phénoliques, à une hydrolyse catalysée par une base .

18. Extrait hydrolysé de farine de graines de lin selon la revendication 17 exempte de glycosides cyanogéniques.
